**Europäisches Patentamt**

**European Patent Office**

**Office européen des brevets**

(19)

(11) Publication number : **0 350 171 B1**

# EUROPEAN PATENT SPECIFICATION

(12)

(45) Date of publication of patent specification : **13.05.92 Bulletin 92/20**

(51) Int. Cl.⁵ : **C07C 2/72**

(21) Application number : **89306041.8**

(22) Date of filing : **14.06.89**

(54) **Coupling process.**

(30) Priority : **17.06.88 US 207959**

(43) Date of publication of application : **10.01.90 Bulletin 90/02**

(45) Publication of the grant of the patent : **13.05.92 Bulletin 92/20**

(84) Designated Contracting States : **CH DE FR GB IT LI**

(56) References cited : **EP-A- 0 173 335**

(73) Proprietor : **ETHYL CORPORATION Ethyl Tower 451 Florida Boulevard Baton Rouge Louisiana 70801 (US)**

(72) Inventor : **Smith, Robert Scott 8978 G.S.R.I. Avenue Baton Rouge Louisiana 70810 (US)**

(74) Representative : **Collier, Jeremy Austin Grey et al J.A.Kemp & Co. 14, South Square Gray's Inn London WC1R 5EU (GB)**

Jouve, 18, rue Saint-Denis, 75001 PARIS

## Description

This invention relates to a process for coupling an alkene with an aromatic hydrocarbon having an active hydrogen on a saturated alpha-carbon.

As disclosed, e.g., in U.S. Patent 4,179,580 (Cobb), European Patent 128001 (Kudoh et al.), and Eberhardt et al., Journal of Organic Chemistry, Vol. 30, pp. 82-84 (1965), it is known that supported alkali metals are useful as catalysts in the coupling of ethylenically-unsaturated hydrocarbons with aromatic hydrocarbons having an active hydrogen on a saturated alpha-carbon. The supported alkali metals are more effective than the corresponding unsupported alkali metals in such reactions but are still not as effective as might be desired.

Claff et al., Journal of Organic Chemistry, Vol. 20, pp. 440-442 and 981-986 (1955) disclose the use of sodium oxide in the metalation of toluene by potassium.

Pines et al., Journal of the American Chemical Society, Vol. 80, pp. 6001-6004 (1958) disclose the use of sodium hydroxide as a promoter for the sodium-catalyzed side chain aralkylation of alkylbenzenes with styrene.

An object of this invention is to provide a novel process for coupling an alkene with an aromatic hydrocarbon having an active hydrogen on a saturated alpha-carbon.

Another object is to provide such a process which utilizes a supported alkali metal as a catalyst.

A further object is to provide such a process in which the reaction rate and product yield are increased.

These and other objects are attained by coupling an alkene with an aromatic hydrocarbon having an active hydrogen on a saturated alpha-carbon in the presence of a supported alkali metal as a catalyst and 5-100 mol %, based on the amount of the alkali metal catalyst, of sodium hydroxide, potassium hydroxide, or a mixture thereof as a co-catalyst.

The alkene which is coupled with the aromatic hydrocarbon in the practice of the invention may be any of the alkenes that are known to be useful in such reactions, such as ethene, propene, 1-butene, 2-butene, isobutene, 1-pentene, 2-pentene, 3-methyl-1-butene, 2-methyl-2-butene, 1-hexene, 2-hexene, 3-hexene, 4-methyl-1-pentene, 3-methyl-1-pentene, 4-methyl-2-pentene, 1-heptene, 2-heptene, 2-octene, 4-nonene, 1-decene, 2-decene, 1-dodecene, 3-tetradecene, 5-hexadecene, 6-methyl-4-heptadecene, and 1-eicosene. However, it is generally an alkene corresponding to the formula $QQ'C=CTT'$, in which Q, Q', T, and T' are independently selected from hydrogen and alkyl groups of up to 20 carbons; and it is apt preferably to be an alkene of up to 20 carbons. Particularly preferred alkenes are ethene and propene.

The aromatic hydrocarbon having an active hydrogen on a saturated alpha-carbon may be any such compound that is known to be useful in such reactions, such as toluene, ethylbenzene, n-propylbenzene, isopropylbenzene, n-butylbenzene, sec-butylbenzene, isobutylbenzene, n-eicosylbenzene, o-, m-, and p-xylenes, o-, m-, and p-ethyltoluenes, 1,3,5-trimethylbenzene, 1,2,4,5- and 1,2,3,5-tetramethylbenzenes, p-diisopropylbenzene, 1- and 2-methylnaphthalenes, dimethylnaphthalenes, 1-ethyl-4-n-octadecylnaphthalene, 1,4-di-n-pentylnaphthalene, 1,2,3,4-tetrahydronaphthalene, indan, cyclohexylbenzene, methylcyclohexylbenzene, and diphenylmethane. However, it is generally a hydrocarbon corresponding to the formula $RR'R''CH$, in which R is an aryl group of up to 20 carbons and R' and R'' are independently selected from hydrogen and alkyl and aryl groups of up to 20 carbons; and it is apt preferably to be an alkylbenzene having one or more ar-alkyl groups. A particularly preferred aromatic hydrocarbon is toluene.

The mol ratio of alkene to aromatic hydrocarbon varies with the particular reactants employed and the products desired, particularly since the aromatic hydrocarbon may have one or more active hydrogens, and it may be desired to react the alkene with only one or with more than one active hydrogen in the aromatic hydrocarbon. It is frequently preferred to employ the reactants in the stoichiometric amounts appropriate for the preparation of the desired product. However, either reactant can be used in excess.

As in Cobb, the alkali metal employed as a catalyst may be lithium, sodium, potassium, rubidium, or cesium; and it appropriately has its surface area increased by being finely divided or liquid as well as by being supported on any suitable support material, such as diatomaceous earth, activated charcoal, granular coke, silica, alumina, pumice, porcelain, quartz, steel turnings, copper shot, sodium carbonate, or potassium carbonate. However, it is preferably potassium or a potassium alloy, e.g., a sodium/potassium alloy. The amount of alkali metal used is a catalytic amount, generally 2-10 mol %, based on the amount of either of the reactants when they are employed in equimolar amounts or on the amount of the major reactant when they are not utilized in equimolar amounts.

The co-catalyst of the invention is sodium hydroxide, potassium hydroxide, or a mixture thereof. Like the alkali metal, it is used in finely divided form; and it may be incorporated into the reaction mixture as the hydroxide, or it may be generated in situ by reacting water with supported sodium and/or potassium. The amount of co-catalyst used is 5-100 mol %, based on the amount of the alkali metal catalyst. When it is generated in situ, as in a preferred embodiment of the invention, it is usually formed by adding 5-50 mol %, preferably 5-25 mol %, of water to a supported sodium and/or potassium catalyst.

The reaction is conducted by heating a mixture of the alkene, the active hydrogen-containing aromatic hydrocarbon, the supported catalyst, and the co-catalyst under substantially anhydrous conditions at a suitable temperature, generally 100-300°C., preferably 175-200°C., to couple the reactants. It is generally conducted in the absence of a diluent or in the presence of an excess of the active hydrogen-containing aromatic hydrocarbon as the sole diluent. However, an inert diluent can be used if desired. Exemplary of such diluents are liquid alkanes, cycloalkanes, and aromatic hydrocarbons, such as pentane, hexane, isooctane, cyclohexane, naphthalene, decahydronaphthalene, and white oils.

When the co-catalyst is to be generated in situ, it is preferred to heat a mixture of the aromatic hydrocarbon and a supported sodium and/or potassium catalyst to the reaction temperature prior to adding water in order to shorten the induction period that sometimes follows the subsequent addition of the alkene.

The process of the invention proceeds at a faster rate and provides higher product yields with fewer by-products than comparable processes conducted in the absence of the co-catalyst, and it is particularly advantageous as a means of alkylating alkylaromatic compounds, especially alkylbenzenes, to form compounds useful as solvents, internal standards, or intermediates for polymers, pharmaceuticals, or pesticides.

The following examples are given to illustrate the invention.

COMPARATIVE EXAMPLE A

A suitable reaction vessel was charged with 4.9g of diatomaceous earth, 92g (1.0 mol) of dry toluene, $C_{11}$ paraffin as a GC standard, and 1.0g of potassium. The mixture was stirred and heated to 185°C. in 15 minutes, after which propene was charged until a pressure of 2758 kPa was reached; and the propene pressure was then maintained at 2689-2758 kPa throughout the reaction. Periodically the stirrer was stopped to allow the solids to settle; and samples were drawn, allowed to cool to room temperature, and subjected to GC analysis to determine the amounts of unreacted toluene, desired isobutylbenzene (IBB) product, and n-butylbenzene (NBB), methylindan (MI), and methylpentene (MP) by-products. Finally the reaction was stopped by cooling the reactor to 75°C., venting most of the propene, and injecting 10 mL of methanol under nitrogen pressure to quench the catalyst. The results of the analyses are shown below.

| Time (min.) | Toluene | IBB | NBB | MI | MP |
|---|---|---|---|---|---|
| 0 | 100 | 0 | 0 | 0 | 0 |
| 40 | 83.0 | 10.0 | 0.42 | 2.60 | 1.0 |
| 80 | 74.2 | 16.7 | 0.73 | 4.05 | 1.8 |
| 120 | 66.8 | 22.0 | 0.94 | 4.97 | 2.5 |
| 160 | 61.2 | 25.1 | 1.09 | 5.45 | 3.3 |
| 200 | 59.0 | 26.9 | 1.11 | 5.72 | 3.4 |

*Mols x 100*

COMPARATIVE EXAMPLE B

Comparative Example A was repeated except that a portion of the dry toluene was replaced with toluene containing 565 ppm of water so as to provide 0.31 mmol of water in the reaction mixture, and the amount of potassium was increased to 1.02g (26.1 mmols) to compensate for the amount of potassium that would react with the water. Thus, the reaction mixture contained about 1.2 mol % of co-catalyst, based on the amount of catalyst. The analytical results are shown below.

| Time (min.) | Mols x 100 | | | | |
|---|---|---|---|---|---|
| | Toluene | IBB | NBB | MI | MP |
| 0 | 100 | 0 | 0 | 0 | 0 |
| 40 | 83.2 | 8.1 | 0.57 | 0.81 | 0.9 |
| 80 | 68.7 | 22.1 | 1.33 | 2.68 | 2.6 |
| 120 | 60.9 | 29.1 | 1.70 | 3.41 | 3.8 |
| 160 | 52.9 | 35.2 | 1.99 | 4.22 | 3.9 |
| 200 | 48.7 | 38.1 | 2.09 | 4.40 | 4.9 |

As demonstrated in the preceding examples, some increases in reaction rate and product yield are achieved by the use of very small amounts of water in the reaction mixture. The following example shows that greater improvements are effected by the use of an amount of water within the scope of the present invention.

EXAMPLE I

Comparative Example B was repeated except that the amount of water in the reaction mixture was increased to 2.9 mmols and the amount of potassium was increased to 1.22g (31.2 mmols). Thus, the reaction mixture contained about 10.2 mol % of co-catalyst, based on the amount of catalyst. The analytical results are shown below.

| Time (min.) | Mols x 100 | | | | |
|---|---|---|---|---|---|
| | Toluene | IBB | NBB | MI | MP |
| 0 | 100 | 0 | 0 | 0 | 0 |
| 40 | 91.3 | 4.1 | 0.41 | 0 | 0.6 |
| 80 | 74.2 | 18.8 | 2.00 | 0.12 | 2.2 |
| 120 | 56.9 | 37.1 | 3.99 | 0.27 | 5.0 |
| 160 | 39.9 | 52.8 | 5.21 | 0.44 | 7.4 |
| 200 | 26.9 | 64.8 | 5.90 | 0.58 | 12.6 |

EXAMPLE II

Comparative Example A was essentially repeated except that the 1.0g of potassium was replaced with 1.9g of NaK (an alloy having a K content of 78% by weight) and 8.6 mmols of water were charged to the reaction vessel before heating was begun. The analytical results are shown below.

| Time (min.) | Mols x 100 | | | | |
|---|---|---|---|---|---|
|  | Toluene | IBB | NBB | MI | MP |
| 0 | 100 | 0 | 0 | 0 | 0 |
| 40 | no samples taken - no propene uptake | | | | |
| 120 | no samples taken - no propene uptake | | | | |
| 160 | 98.3 | 1.4 | 0.14 | 0 | 0.1 |
| 200 | 84.1 | 12.6 | 1.65 | 0.23 | 2.0 |
| 240 | 62.3 | 33.5 | 4.06 | 0.58 | 3.5 |
| 280 | 30.4 | 53.7 | 5.94 | 0.76 | 5.4 |
| 320 | 19.5 | 64.2 | 6.56 | 0.80 | 8.7 |
| 360 | 13.0 | 70.0 | 6.54 | 0.76 | 11.7 |

EXAMPLE III

Example II was repeated except that the water was not added until the mixture of diatomaceous earth, toluene, paraffin, and NaK had been heated to 185°C. The analytical results are shown below.

| Time (min.) | Mols x 100 | | | | |
|---|---|---|---|---|---|
|  | Toluene | IBB | NBB | MI | MP |
| 0 | 100 | 0 | 0 | 0 | 0 |
| 40 | 100 | 0 | 0 | 0 | 0 |
| 80 | 100 | 0 | 0 | 0 | 0 |
| 120 | 89.8 | 7.0 | 0.82 | 0.13 | 0.7 |
| 160 | 62.8 | 22.7 | 2.79 | 0.49 | 2.7 |
| 200 | 44.0 | 43.4 | 4.92 | 0.77 | 5.8 |
| 240 | 26.5 | 58.2 | 6.05 | 0.88 | 8.9 |
| 280 | 16.3 | 67.2 | 6.37 | 0.89 | 12.4 |

COMPARATIVE EXAMPLE C

Example III was essentially repeated except that no diatomaceous earth or other support was employed. The analytical results are shown below.

5

| Time (min.) | Mols x 100 | | | | |
|---|---|---|---|---|---|
| | Toluene | IBB | NBB | MI | MP |
| 90 | 100 | 0 | 0 | 0 | 0 |
| 160 | 97.5 | 1.2 | 0.15 | 0 | 0.1 |
| 200 | 92.9 | 2.7 | 0.34 | 0 | 0.3 |
| 240 | 88.4 | 5.7 | 0.71 | 0.03 | 0.5 |

COMPARATIVE EXAMPLE D

A suitable reaction vessel was charged with 92g of toluene, 4.9g of -325 mesh potassium carbonate, 1.0g of NaK, and $C_{11}$ paraffin as an internal standard. The mixture was heated to 185°C. with stirring, and propene was fed to a pressure of 2758 kPa, a pressure that was maintained. Samples were taken periodically and the reaction was then stopped as in Comparative Example A. The analytical results are shown below.

| Time (min.) | Mols x 100 | | | | |
|---|---|---|---|---|---|
| | Toluene | IBB | NBB | MI | MP |
| 0 | 100 | 0 | 0 | 0 | 0 |
| 120 | 95.2 | 2.2 | 0.25 | 0.06 | 0.4 |
| 160 | 87.1 | 10.4 | 0.97 | 0.56 | 1.3 |
| 200 | 70.7 | 22.9 | 1.93 | 1.16 | 3.3 |
| 240 | 70.5 | 23.5 | 1.84 | 1.43 | 3.0 |
| 280 | 67.4 | 26.6 | 1.97 | 1.69 | 3.8 |

EXAMPLE IV

Comparative Example D was repeated except that 0.26g of -325 mesh sodium hydroxide was included in the initial charge to the reaction vessel. The analytical results are shown below.

| Time (min.) | Mols x 100 | | | | |
|---|---|---|---|---|---|
| | Toluene | IBB | NBB | MI | MP |
| 0 | 100 | 0 | 0 | 0 | 0 |
| 120 | 94.9 | 1.2 | 0.16 | 0.01 | 0.1 |
| 200 | 81.3 | 12.9 | 1.79 | 0.13 | 1.6 |
| 240 | 65.5 | 26.1 | 3.62 | 0.19 | 3.2 |
| 280 | 48.8 | 39.2 | 5.38 | 0.22 | 5.1 |
| 320 | 36.2 | 51.2 | 6.76 | 0.18 | 8.0 |

## Claims

1. A process for coupling an alkene with an aromatic hydrocarbon having an active hydrogen on a saturated alpha-carbon which comprises contacting the said alkene and aromatic hydrocarbon with a supported alkali metal as catalyst, and as co-catalyst 5-100 mol %, based on the amount of the alkali metal catalyst, of sodium hydroxide, potassium hydroxide, or a mixture thereof.

2. Process according to claim 1 wherein the alkene is propene and the aromatic hydrocarbon is toluene.

3. Process according to claim 1 or 2 wherein the alkali metal catalyst is potassium or a potassium alloy.

4. Process according to claim 3 wherein the alkali metal catalyst is sodium/potassium.

5. Process according to any one of claims 1 to 4 wherein the co-catalyst is a preformed hydroxide or mixture of hydroxides.

6. Process according to any one of claims 1 to 4 wherein the co-catalyst is a hydroxide or hydroxide mixture formed in situ by adding water to a reaction mixture containing a supported sodium and/or potassium catalyst.

7. Process according to claim 6 wherein the catalyst is a supported potassium or sodium/potassium catalyst.

8. Process according to any one of claims 1 to 7 which is conducted at a temperature of 100-300°C.

9. Process according to claim 8 wherein the reaction temperature is 175-200°C.

10. Process according to claim 1 wherein propene is coupled with toluene at 175-200°C in the presence of a supported potassium or sodium/potassium catalyst and a co-catalyst formed in situ by adding water to the catalyst prior to contacting the reactants.

## Patentansprüche

1. Verfahren zur Kupplung eines Alkens mit einem aromatischen Kohlenwasserstoff mit einem aktiven Wasserstoff an einem gesättigten α-Kohlenstoffatom, welches das In-Kontakt-Bringen des Alkens und des aromatischen Kohlenwasserstoffs mit einem Träger-gestützten Alkalimetall als Katalysator und als Co-Katalysator mit 5 bis 100 Molprozent Natriumhydroxid, Kaliumhydroxid oder einer Mischung daraus, bezogen auf die Menge an Alkalimetall-Katalysator, umfaßt.

2. Verfahren nach Anspruch 1, worin das Alken Propen ist und der aromatische Kohlenwasserstoff Toluol ist.

3. Verfahren nach Anspruch 1 oder 2, worin der Alkalimetall-Katalysator Kalium oder eine Kalium-Legierung ist.

4. Verfahren nach Anspruch 3, worin der Alkalimetall-Katalysator Natrium/Kalium ist.

5. Verfahren nach irgendeinem der Ansprüche 1 bis 4, worin der Co-Katalysator ein vorher gebildetes Hydroxid oder eine vorher gebildete Mischung aus Hydroxiden ist.

6. Verfahren nach irgendeinem der Ansprüche 1 bis 4, worin der Co-Katalysator ein Hydroxid oder eine Mischung von Hydroxiden ist, die in situ durch Zusatz von Wasser zu einer Reaktionsmischung gebildet wird, die einen Träger-gestützten Natrium-und/oder Kalium-Katalysator enthält.

7. Verfahren nach Anspruch 6, worin der Katalysator ein Träger-gestützter Kalium-oder Natrium/Kalium-Katalysator ist.

8. Verfahren nach irgendeinem der Ansprüche 1 bis 7, welches bei einer Temperatur von 100 bis 300°C durchgeführt wird.

9. Verfahren nach Anspruch 8, worin die Reaktionstemperatur 175 bis 200°C beträgt.

10. Verfahren nach Anspruch 1, worin Propen mit Toluol bei 175 bis 200°C in Gegenwart eines Träger-gestützten Kalium- oder Natrium/Kalium-Katalysators und eines Co-Katalysators gekuppelt wird, der in situ durch Zusatz von Wasser zu dem Katalysator vor dem Kontakt mit den Reaktanden gebildet wird.

## Revendications

1. Procédé de couplage d'un alcène avec un hydrocarbure aromatique portant un atome d'hydrogène actif sur un atome alpha saturé de carbone, qui consiste à faire entrer ledit alcène et ledit hydrocarbure aromatique en contact avec un métal alcalin fixé sur un support comme catalyseur, et comme co-catalyseur, 5 à 100 moles %, sur la base de la quantité de catalyseur au métal alcalin, d'hydroxyde de sodium, d'hydroxyde de potassium ou d'un mélange des deux.

2. Procédé suivant la revendication 1, dans lequel l'alcène est le propène et l'hydrocarbure aromatique est le toluène.

7

3. Procédé suivant la revendication 1 ou 2, dans lequel le catalyseur à base de métal alcalin est le potassium ou un alliage de potassium.

4. Procédé suivant la revendication 3, dans lequel le catalyseur à base de métal alcalin est le sodium/potassium.

5. Procédé suivant l'une quelconque des revendications 1 à 4, dans lequel le co-catalyseur est un hydroxyde préformé ou un mélange d'hydroxydes.

6. Procédé suivant l'une quelconque des revendications 1 à 4, dans lequel le co-catalyseur est un hydroxyde ou un mélange d'hydroxydes formé in situ par addition d'eau à un mélange réactionnel contenant un catalyseur au sodium et/ou au potassium fixé sur un support.

7. Procédé suivant la revendication 6, dans lequel le catalyseur est un catalyseur au potassium ou au sodium/potassium fixé sur un support.

8. Procédé suivant l'une quelconque des revendications 1 à 7, qui est mis en oeuvre à une température de 100 à 300°C.

9. Procédé suivant la revendication 8, dans lequel la température de réaction va de 175 à 200°C.

10. Procédé suivant la revendication 1, dans lequel du propène est couplé à du toluène à 175-200°C en présence d'un catalyseur au potassium ou au sodium/potassium fixé sur un support et d'un co-catalyseur formé in situ par addition d'eau au catalyseur avant la mise en contact des corps réactionnels.